# EUROPEAN PATENT SPECIFICATION

(11) **EP 2 006 390 B1**
(45) Date of publication and mention of the grant of the patent: **19.07.2017**
(21) Application number: 07739984.8
(22) Date of filing: 28.03.2007
(51) Int. Cl.: C12P 13/02, C07C 231/24, C07C 233/09, C08F 20/56, C08F 220/56

(54) **PROCESS FOR PRODUCTION OF ACRYLAMIDE**
VERFAHREN ZUR HERSTELLUNG VON ACRYLAMID
PROCÉDÉ DE PRODUCTION D'ACRYLAMIDE

(30) Priority: 06.04.2006 JP 2006105463
(43) Date of publication of application: 24.12.2008
(73) Proprietor: Mitsui Chemicals, Inc., Tokyo 105-7117 (JP)
(72) Inventor: ABE, Takeya MITSUI CHEMICALS, INC.,, Takaishi-shi, Osaka 592-8501 (JP); FUKUDA, Takeshi MITSUI CHEMICALS, INC.,, Takaishi-shi, Osaka 592-8501 (JP); MURAMOTO, Masanori MITSUI CHEMICALS, INC.,, Takaishi-shi, Osaka 592-8501 (JP); HAZAMA, Souichi MITSUI CHEMICALS, INC.,, Takaishi-shi, Osaka 592-8501 (JP)
(74) Representative: Wytenburg, Wilhelmus Johannes
(86) International application number: PCT/JP2007/056546
(87) International publication number: WO 2007/116781

(56) References cited:
- EP-A1- 0 670 301
- WO-A1-01/53253
- WO-A1-01/73101
- WO-A1-83/01784
- GB-A- 2 061 977
- JP-A- 2000 015 113
- JP-A- 2000 016 978
- JP-A- 2002 145 963
- US-A- 4 001 081
- US-A- 5 534 655
- DEB ET AL: "Uncatalysed Knoevenagel condensation in aqueous medium at room temperature", TETRAHEDRON LETTERS, ELSEVIER, AMSTERDAM, NL, vol. 46, no. 38, 19 September 2005 (2005-09-19), pages 6453-6456, XP005034740, ISSN: 0040-4039, DOI: 10.1016/J.TETLET.2005.07.111
- KLAVINS M ET AL: "Catalytic activity of humic substances in condensation reactions", ENVIRONMENT INTERNATIONAL, PERGAMON PRESS, US, vol. 24, no. 5-6, 1 January 1998 (1998-01-01), pages 645-651, XP003018260, ISSN: 0160-4120, DOI: 10.1016/S0160-4120(98)00032-4

## Description

### TECHNICAL FIELD

The present invention relates to a process for producing acrylamide with improved quality and a process for producing an acrylamide-based polymer using this acrylamide with improved quality. More particularly, the invention relates to a process for producing acrylamide with improved quality by adding at least one compound selected from dimedone, barbituric acid, hydantoin, and salts thereof to acrylamide which is obtained by hydrating acrylonitrile using a bacterial cell containing nitrile hydratase and a substance obtained by treating the bacterial cell, and a process for producing an acrylamide-based polymer using this acrylamide.

### BACKGROUND ART

Acrylamide is mainly used as a raw material of an acrylamide-based polymer, and in recent years, this acrylamide-based polymer has been desired to have much higher quality. For example, with the recent demand for enhancement of performance, an acrylamide-based polymer used as a coagulant has been desired to have a much higher molecular weight while maintaining water solubility and excellent color tone.

On that account, it has been required that impurities contained in trace amounts in acrylamide, which are considered to have evil influence on the quality of an acrylamide-based polymer such as water solubility when the acrylamide-based polymer is produced from the acrylamide, should be made harmless much more accurately.

As such impurities having evil influence, aldehydes, such as acrolein contained in acrylonitrile that is a raw material of acrylamide, are known. As a means to remove evil influence exerted by this acrolein or the like from acrylamide, for example, a method of bringing acrylamide into contact with a porous ion-exchange resin having a primary and/or a secondary amino group as an exchange group to remove acrolein contained in acrylonitrile (see for example patent document 1) or a method of bringing acrylamide into contact with a gel type weakly basic ion-exchange resin having a primary and/or a secondary amino functional group to decrease aldehydes contained in acrylonitrile, substantially acrolein (see for example patent document 2), is known.

In the above removal methods, however, equipment for purifying acrylontrile by the ion-exchange resin is newly required. Further, since the ion-exchange resin has its limits in performance, acrolein cannot be always removed sufficiently in some cases. Furthermore, if acrolein is tried to be industrially sufficiently removed by the ion-exchange resin only, the purifying process bears a severe burden, and productivity and production cost are sometimes deteriorated.

Instead of the above methods, a method of allowing an anion-exchange resin to support thereon a compound having an active methylene group and an acidic group, such as malonic acid, a malonic acid monoester or cyanoacetic acid, to remove aldehydes contained in acrylonitrile, such as acrolein (see for example patent document 3), or a method comprising introducing a compound having an active methylene group and an acidic group into a reactor for synthesizing acrylamide from acrylonitrile by the use of a copper catalyst, performing reaction with aldehydes such as acrolein and then removing the reaction products by the use of an ion-exchange resin or the like (see for example patent document4) is known.

In the above methods, however, equipment for allowing the ion-exchange resin to support the compound or equipment for removing aldehydes from the reaction solution is newly required, similarly to the aforesaid methods, and an increase of equipment cost cannot be avoided. According to these methods, it is feasible to make acrolein harmless to a certain extent, but from the viewpoint that acrolein is made harmless and the viewpoint that water solubility and excellent color tone of the polymer obtained using the acrylamide as a raw material are maintained with increasing the molecular weight of the polymer, there is yet room for improvement.
Patent document 1: Japanese Patent Publication No. 1108/1983
Patent document 2: Japanese Patent Laid-Open Publication No. 134063/1983
Patent document 3: Japanese Patent Laid-Open Publication No. 15113/2000
Patent document 4: Japanese Patent Laid-Open Publication No. 291907/1995

US-A-4001081, EP-A-93782, EP-A-1182260 and EP-A-1167345 disclose the preparation of acrylamide from acrylonitrile with the action of a nitrile hydratase.

GB-A-2069977 teaches the addition of a compound with an 1,3-dione moiety, e.g. barbituric acid and dimedone, to acrylamide prepared with copper catalysts in order to improve the water-solubility of acrylamide polymers.

### DISCLOSURE OF THE INVENTION

### PROBLEM TO BE SOLVED BY THE INVENTION

It is an object of the present invention to provide a process for producing acrylamide with improved quality by adding a specific substance to acrylamide which is obtained by hydrating acrylonitrile using a bacterial cell containing nitrile hydratase or a substance obtained by treating the bacterial cell, and a process for producing an acrylamide-based polymer wherein even if an acrylamide-based polymer having a high molecular weight is produced using the above acrylamide, a polymer excellent in water solubility and color tone can be obtained.

### MEANS TO SOLVE THE PROBLEM

The present inventors have studied the aforesaid problems. As a result, they have found that high-quality acrylamide is obtained by adding at least one compound selected from the group consisting of dimedone, barbituric acid, hydantoin and salts thereof which is obtained by hydrating acrylonitrile using a bacterial cell containing nitrile hydratase or a substance obtained by treating the bacterial cell and that even if a polymer having a high molecular weight is produced from this acrylamide, an acrylamide-based polymer excellent in qualities such as water solubility and color tone can be obtained, and they have accomplished the present invention.

That is to say, the process for producing acrylamide of the present invention is characterized by adding at least one compound selected from the group consisting of dimedone, barbituric acid, hydantoin and salts thereof to acrylamide and the acrylamide is prepared by hydrating acrylonitrile using a bacterial cell containing nitrile hydratase or a substance obtainable by treating the bacterial cell. The acrylamide-based polymer of the present invention is prepared by homopolymerizing the above acrylamide or copolymerizing the above acrylamide and at least one unsaturated monomer copolymerizable with the acrylamide.

### EFFECT OF THE INVENTION

According to the present invention, high-quality acrylamide can be obtained, and even if a polymer having a high molecular weight is produced using this acrylamide, an acrylamide-based polymer excellent in qualities such as water solubility and color tone can be obtained.

### BEST MODE FOR CARRYING OUT THE INVENTION

The present invention is described in detail hereinafter.

### Acrylamide

In the method of the invention, the acrylamide is prepared by hydrating acrylonitrile using, as a catalyst, a microorganism bacterial cell containing nitrile hydratase or a substance obtained by treating the bacterial cell. In general, the acrylamide obtained in this manner contains smaller amounts of impurities and does not have evil influence on polymerization of acrylamide, as compared with, for example, acrylamide obtained by catalytic hydration of acrylonitrile using a copper catalyst. As for improvement in quality and quality of a polymer obtained by polymerizing the acrylamide, however, problems sometimes occur.

In the present invention, nitrile hydratase means an enzyme having an ability to hydrolyze a nitrile compound to form the corresponding amide compound. The microorganism containing nitrile hydratase referred to herein is not specifically restricted as long as it is a microorganism which produces nitrile hydratase having an ability to hydrolyze a nitrile compound to form the corresponding amide compound and retains activity of nitrile hydratase in an acrylamide aqueous solution.

Preferred examples of the microorganisms include those belonging to genus Nocardia, genus Corynebacterium, genus Bacillus, themophilic genus Bacillus, genus Pseudomonas, genus Micrococcus, genus Rhodococcus represented by rhodochrous species, genus Acinetobacter, genus Xanthobacter, genus Streptomyces, genus Rhizobium, genus Klebsiella, genus Enterobacter, genus Erwinia, genus Aeromonas, genus Citrobacter, genus Acromobacter, genus Agrobacterium, and genus Pseudonocardia represented by thermophila species.

In the microorganisms referred to in the invention, transformants obtained by expressing nitrile hydratase genes having been cloned from the above microorganisms in arbitrary hosts are also included. A typical example of the arbitrary host is Escherichia coli, as shown in the later-described example. However, the arbitrary host is not limited to the Escherichia coli, and strains of other microorganisms such as bacteria of genus Bacillus (specifically Bacillus subtilis), yeast and actinomycetes are also included. An example of such strain is MT-10822, and this strain has been deposited with an accession number of FERM BP-5785 to National Institute of Bioscience and Human-Technology, Agency of Industrial Science and Technology, the Ministry of International Trade and Industry, 1-3, Higashi 1-chome, Tsukuba-shi, Ibaraki-ken (present: International Patent Organism Depositary, National Institute of Advanced Industrial Science and Technology, Independent Administrative Institution, Tsukuba Central 6, 1-1-1, Higashi, Tsukuba-shi, Ibaraki-ken) on February 7, 1996, under the Budapest treaty on the International Recognition of the Deposit of Microorganisms for the Purpose of Patent Procedure. Further, transformants wherein variant type nitrile hydratase having been much more improved in acrylamide resistance, acrylonitrile resistance and temperature resistance is expressed by means of replacement of one or more constituent amino acids of the enzyme with other amino acids, deficiency thereof, deletion thereof or insertion thereof utilizing recombinant DNA technology are also included in the microorganisms referred to in the invention.

In the production of an amide compound using such a microorganism as mentioned above, a bacterial cell of the microorganism or a substance obtained by treating the bacterial cell is usually used. The bacterial cell is prepared by general methods publicly known in fields of molecular biology, biological engineering and gene engineering. For example, there can be mentioned a method comprising culturing the microorganism in a usual liquid culture medium such as LB culture medium or M9 culture medium, then growing it at an appropriate culture temperature (it is generally 20°C to 50°C, but it may be not lower than 50°C in the case of thermophilic bacteria), subsequently separating the microorganism from the culture solution by centrifugation and recovering the microorganism.

The substance obtained by treating a bacterial cell of a microorganism in the invention means an extract or a ground substance of the microorganism bacterial cell, a separated substance obtained by separating and purifying a nitrile hydratase active fraction of the extract or the ground substance, a fixed substance obtained by fixing the microorganism bacterial cell or an extract, a ground substance or a separated substance of the bacterial cell onto a proper carrier, or the like, and any of them corresponds to the substance obtained by treating a bacterial cell of the invention as long as it has activity of nitrile hydratase. Of such substances, a single substance may be used, or two or more substances different in shape may be used simultaneously or alternately.

### Additive compound and salt

In the present invention, at least one compound selected from the group consisting of dimedone, barbituric acid, hydantoin and salts thereof.

When such a compound is added to the acrylamide, an acrylamide-based polymer having a much higher molecular weight can be obtained, and besides the acrylamide-based polymer has much more excellent water solubility.

In the present invention, the above compound and the above salt can be used in combination. Further, the above compounds may be used singly or in combination, and the above salts may be used singly or in combination.

The amounts of the compound and the salt added are not specifically restricted, but in order to obtain acrylamide of excellent quality and in order that the acrylamide purifying process should not bear an excess burden, the amount of the compound present in the solution is in the range of usually 10 to 10000 ppm by weight, preferably 50 to 5000 ppm by weight, based on the whole weight of the reaction solution.

For adding the compound a method of directly adding the compound to an acrylamide aqueous solution, a method comprising dissolving the compound in a small amount of water and adding the solution, etc. are available.

When the acrylamide obtained as above is homopolymerized or copolymerized with another monomer to prepare an acrylamide-based polymer, the resulting polymer not only has a sufficiently high molecular weight but also shows markedly improved water solubility and excellent color tone.

### Production of acrylamide-baled polymer

The acrylamide produced in the first method of the invention can be homopolymerized or copolymerized with other monomers.

Examples of the other monomers copolymerizable with the acrylamide include:
unsaturated carboxylic acids, such as acrylic acid, methacrylic acid, itaconic acid, maleic acid and fumaric acid, and salts thereof;
vinylsulfonic acid, styrenesulfonic acid, acrylamidomethylpropanesulfonic acid, and salts thereof;
alkylaminoalkyl esters of (meth)acrylic acids, such as N,N-dimethylaminoethyl methacrylate, N,N-diethylaminoethyl methacrylate and N,N-dimethylaminoethyl acrylate, and quaternary ammonium derivatives thereof;
N,N-dialkylaminoalkyl (meth)acrylamides, such as N,N-dimethylaminopropyl methacrylamide and N,N-dimethylaminopropyl acrylamide, and quaternary ammonium derivatives thereof;
hydrophilic acrylamides, such as acetone acrylamide, N,N-dimethyl acrylamide, N,N-dimethyl methacrylamide, N-ethyl methacrylamide, N-ethyl acrylamide, N,N-diethyl acrylamide and N-propyl acrylamide;
N-acryloylpyrrolidine, N-acryloylpiperidine and N-acryloylmorpholine;
hydroxyethyl methacrylate, hydroxyethyl acrylate, hydroxypropyl methacrylate and hydroxypropyl acrylate;
methoxypolyethylene glycol (meth)acrylate and N-vinyl-2-pyrrolidone;
N-alkyl (meth)acrylamide derivatives, such as N,N-di-n-propyl acrylamide, N-n-butyl acrylamide, N-n-hexyl acrylamide, N-n-hexyl methacrylamide, N-n-octyl acrylamide, N-n-octyl methacrylamide, N-tert-octyl acrylamide, N-dodecyl acrylamide and N-n-dodecyl methacrylamide;
N-(ω-glycidoxyalkyl)(meth)acrylamide derivatives, such as N,N-diglycidyl acrylamide, N,N-diglycidyl methacrylamide, N-(4-glycidoxybutyl)acrylamide, N-(4-glycidoxybutyl)methacrylamide, N-(5-glycidoxypentyl)acrylamide and N-(6-glycidoxyhexyl)acrylamide;
(meth)acrylate derivatives, such as methyl (meth)acrylate, ethyl (meth)acrylate, butyl (meth)acrylate, lauryl (meth)acrylate, 2-ethylhexyl (meth)acrylate and glycidyl (meth)acrylate; and
acrylonitrile, methacrylonitrile, vinyl acetate, vinyl chloride, vinylidene chloride, olefins, such as ethylene, propylene and butene, styrene, α-methylstyrene, butadiene, isoprene and methacrylamide.

The above-mentioned other monomers may be used singly or in combination of two or more kinds.

In the case where the acrylamide and other monomers are copolymerized, their mixing ratio is not specifically restricted, but in usual, other monomers are used in amounts of not more than 100 mol, preferably not more than 50 mol, based on 100 mol of the acrylamide.

Examples of methods for polymerizing the monomers include aqueous solution polymerization and emulsion polymerization.

In the case of the aqueous solution polymerization, the total concentration of the acrylamide and other monomers that are added if necessary is in the range of usually 5 to 90% by weight.

As a polymerization initiator, for example, a radical polymerization initiator is employable.

Examples of the radical polymerization initiators include peroxides, such as potassium persulfate, ammonium persulfate, hydrogen peroxide and benzoyl peroxide; azo type free-radical initiators, such as azobisisobutyronitrile, 2,2'-azobis(4-amidinopropane)dihydrochloride and 4,4'-azobis(sodium 4-cyanovalerianate); redox type catalysts using combination of the above peroxides and reducing agents such as sodium bisulfite, triethanolamine and ammonium ferrous sulfate; and dimethylaminopropionitrile.

The above polymerization initiators may be used singly or may be used in combination. The amount of the polymerization initiator is in the range of usually 0.001 to 5% by weight based on the total weight of the monomers.

The polymerization temperature is in the range of usually -10 to 120°C, more preferably 0 to 90°C. The polymerization temperature does not need to be always maintained at a given temperature, and it may be properly changed with the progress of polymerization. In usual, with the progress of polymerization, heat of polymerization is generated, and the polymerization temperature tends to rise, so that the polymerization system is sometimes cooled when necessary.

The atmosphere in the polymerization is not specifically restricted, but from the viewpoint of a rapid progress of polymerization, polymerization is preferably carried out in an atmosphere of an inert gas such as a nitrogen gas.

Although the polymerization time is not specifically restricted, it is in the range of usually 1 to 20 hours.

Although pH of the aqueous solution in the polymerization is not specifically restricted either, pH may be controlled to carry out polymerization, when needed. Examples of pH adjustors employable in this case include alkalis, such as sodium hydroxide, potassium hydroxide and ammonia; mineral acids, such as phosphoric acid, sulfuric acid and hydrochloric acid; and organic acids, such as formic acid and acetic acid.

Although the molecular weight of the polymer obtained by the invention is not specifically restricted, it is in the range of usually 100,000 to 50,000,000, preferably 500,000 to 30,000,000.

The acrylamide-based polymer obtained in the above manner has markedly improved water solubility, has a sufficiently high molecular weight and is excellent also in color tone. Accordingly, this acrylaimde-based polymer can be favorably used as a coagulant, an additive for paper manufacturing, a petroleum recovering agent or the like.

### EXAMPLES

The present invention is further described with reference to the following examples, but it should be construed that the invention is in no way limited to those examples. Unless otherwise noted, "%" is given on the weight basis.

### Example 1

### Preparation of acrylamide

### Culture of bacterial cell containing nitrile hydratase

A No. 3 clone bacterial cell was obtained in accordance with a method described in Example 1 of Japanese Patent Laid-Open Publication No. 340091/2001, and the bacterial cell was cultured by the method of the same Example 1 to obtain a wet bacterial cell containing nitrile hydratase.

### Preparation of acrylamide

A 1-liter glass flask equipped with a stirrer was prepared as a first reactor, and a Teflon (registered trademark) tube having an inner diameter of 5 mm and a length of 40 m was prepared as a second reactor. In the first reactor, 400 g of water was placed in advance.

The wet bacterial cell obtained by the above culture method was suspended in pure water so that the concentration would become 12%. This suspension was continuously fed to the first reactor at a rate of 11 g/hr with stirring the contents of the reactor. Then, acrylonitrile containing 30 ppm by weight of oxazole and 2 ppm by weight of acrolein was continuously fed at a rate of 31 g/hr, and pure water was continuously fed at a rate of 38 g/hr. Further, a 0.1M-NaOH aqueous solution was continuously fed so that the reaction pH would become 7.5 to 8.5. These raw materials were fed through independent lines from their respective reservoirs, and they did not contact with one another until they were fed into the reactor. In order to keep the liquid level in the first reactor constant, the reaction solution was continuously drawn out of the first reactor at a rate of 80 g/hr and continuously fed to the second reactor, and in the second reactor, the reaction was further promoted.

The first reactor and the second reactor were both immersed in a water bath at a temperature of 10 to 20°C, and temperature control was carried out so that the solution temperature in each of the reactors would become 15°C.

On the second day from the beginning of operation, the reaction solution in each reactor was sampled, and the sample was analyzed by HPLC. As a result, the conversion of acrylonitrile to acrylamide at the outlet of the first reactor was 87%, and the acrylonitrile concentration to the resulting acrylamide at the outlet of the second reactor was not more than the detection limit (not more than 100 ppm by weight,). After the reaction solution was treated with activated carbon in an acidic atmosphere (pH: 5), the wet bacterial cell was removed, and the resulting solution was neutralized with 1N-NaOH to obtain an acrylamide aqueous solution of about 50% by weight.

### Evaluation of polymer quality

### Example 2

To the acrylamide aqueous solution obtained in Example 1, 100 ppm by weight of barbituric acid was added, and then water was added to prepare an acrylamide aqueous solution having a concentration of 20% by weight. In a 1-liter polyethylene container, 500 g of this 20 wt% acrylamide aqueous solution was placed. Then, dissolved oxygen in the solution was removed through nitrogen with maintaining the temperature at 18°C, and immediately, the container was placed in a heat insulating block made of polystyrene foam.

Subsequently, 200×10⁻⁶ mpm (molar ratio to acrylamide) of 4,4'-azobis(sodium 4-cyanovalerianate), 200×10⁻⁶ mpm of dimethylaminopropionitrile and 80×10⁻⁶ mpm of ammonium persulfate were each dissolved in a small amount of water, and they were rapidly poured in this order into the 1-liter polyethylene container. Through these reagents, a nitrogen gas had been passed in advance, and at the time of pouring and before and after the pouring, a small amount of a nitrogen gas was passed through the polyethylene container to prevent inclusion of an oxygen gas.

When the reagents were poured, it was noticed that the temperature inside the polyethylene container rose after the induction time of several minutes, so that nitrogen gas supply was stopped. In the heat insulating block, the polyethylene container was held as it is for about 100 minutes, and as a result, the temperature inside the polyethylene container reached about 70°C. Then, the polyethylene container was taken out of the heat insulating block and immersed in water at 97°C for 2 hours to further promote the polymerization reaction. Thereafter, the container was immersed in cold water to cool it, whereby the polymerization reaction was terminated.

A hydrous gel of an acrylamide polymer obtained as above was taken out of the polyethylene container, then divided into small masses and ground by a mincing machine. The thus ground hydrous gel of an acrylamide polymer was dried with hot air at 100°C for 2 hours and then further pulverized by a high-speed rotary blade pulverizer to obtain a dry powdery acrylamide polymer. The resulting dry powdery acrylamide polymer was sieved to obtain fractions of 32 to 42 meshes. The thus obtained acrylamide polymer was evaluated by the later-described test method for acrylamide polymer. The results are set forth in Table 1.

### Example 3

A polymer sample was obtained by carrying out the same operations as in Example 2, except that to the acrylamide aqueous solution obtained in Example 1 was added 100 ppm by weight of dimedone instead of barbituric acid.

The resulting dry powdery acrylamide polymer was sieved to obtain fractions of 32 to 42 meshes. The thus obtained acrylamide polymer was evaluated by the later-described test method for acrylamide polymer. The results are set forth in Table 1.

### Example 4

A polymer sample was obtained by carrying out the same operations as in Example 25, except that to the acrylamide aqueous solution obtained in Example 1 was added 500 ppm by weight of hydantoin instead of barbituric acid.

The resulting dry powdery acrylamide polymer was sieved to obtain fractions of 32 to 42 meshes. The thus obtained acrylamide polymer was evaluated by the later-described test method for acrylamide polymer. The results are set forth in Table 1.

### Comparative Example 1

A polymer sample was obtained by carrying out the same operations as in Example 2, except that no barbituric acid was added to the acrylamide aqueous solution obtained in Example 1.

The resulting dry powdery acrylamide polymer was sieved to obtain fractions of 32 to 42 meshes. The thus obtained acrylamide polymer was evaluated by the later-described test method for acrylamide polymer. The results are set forth in Table 1.

### Comparative Example 2

### Addition of malonic acid (salt)

The same operations as in Example 2 were carried out, except that to the acrylamide aqueous solution obtained in Example 1 was added 100 ppm by weight of malonic acid instead of barbituric acid. However, the polymerization reaction did not proceed, and any polymer sample was not obtained.

### Reference Example 1

### Preparation of acrylamide by copper catalyst

### Catalyst for hydration reaction

A Raney copper alloy of not more than 80 meshes was developed by a conventional method using caustic soda and cleaned to prepare a Raney copper catalyst. In the preparation of the catalyst and the subsequent handling thereof, contact of the catalyst with an oxygen-containing gas such as air was avoided.

### Catalytic hydration reaction

In an about 2-litter SUS reactor incorporating a stirrer and a catalyst separator, 400 g of the catalyst was placed. To the reactor, acrylonitrile (the same lot product as in Example 1), from which dissolved oxygen had been removed in advance by the use of a nitrogen gas, and water were fed at rates of 600 g/hr and 900 g/hr, respectively, and they were allowed to react with each other at 120°C. The reaction solution was stirred together with the catalyst to give a suspension, and then the suspension was passed through the catalyst separator and taken out of the reactor as a solution hardly containing the catalyst. This reaction was continued for 3 days.

### Concentration

The resulting reaction solution was subjected to vacuum concentration of batch type to distill off the whole amount of unreacted acrylonitrile and a part of unreacted water, whereby an acrylamide aqueous solution having a concentration of about 50% by weight was obtained. The acrylamide aqueous solution contained a copper ion and acrylic acid.

Then, the copper ion and acrylic acid contained were removed in the following manner.

### Purification

A glass column was charged with 150 ml of a strongly acidic cation resin Lewatit SP-112 (trade name, available from Bayer AG) having been pretreated with dilute hydrochloric acid by a conventional method. Further, a glass column was charged with 300 ml of a weakly basic anion resin Lewatit MP-64 (trade name, available from Bayer AG) having been pretreated with a caustic soda aqueous solution by a conventional method. Through these ion-exchange resins, the aforesaid acrylaimide aqueous solution of about 50% by weight was passed at a rate of 900 ml/hr in the order of (1) the strongly acidic cation-exchange resin and (2) the weakly basic anion-exchange resin. The resulting solution had a copper content of less than 0.01 ppm by weight and an acrylic acid content of less than 1 ppm by weight.

The acrylamide aqueous solution of about 50% by weight obtained as above was adjusted to pH 7.0 by the use of caustic soda and sulfuric acid.

### Comparative Example 3

To the acrylamide aqueous solution obtained in Reference Example 1, dimedone was added so that the dimedone content would become 100 ppm by weight, and then the same operations as in Example 2 were carried out to obtain a polymer sample.

The resulting dry powdery acrylamide polymer was sieved to obtain fractions of 32 to 42 meshes. The thus obtained acrylamide polymer was evaluated by the later-described test method for acrylamide polymer. The results are set forth in Table 1.

### Test method for acrylamide polymer

Evaluation of water solubility, measurement of standard viscosity and evaluation of color tone of the polymer samples obtained in Examples 2, 3 and 4 and Comparative Examples 1 and 3 were carried out by the following methods.

### Water solubility

In 1-liter beaker, 600 ml of water was placed, and 0.66 g (purity: 0.6 g) of the polymer sample was added with stirring by a stirring blade of a given shape at 25°C, followed by stirring at 400 rpm for 2 hours. The resulting solution was filtered through a wire cloth of 150 meshes, and from the quantities of insoluble matters and filtering property, water solubility was judged. That is to say, a polymer sample completely dissolved was ranked as AA, a polymer sample nearly completely dissolved was ranked as BB, a polymer sample containing insoluble matters capable of being filtered off was ranked as CC, and a polymer sample containing insoluble matters actually incapable of being filtered because of slow passing of filtrate was ranked as DD. Standard viscosity

The filtrate obtained by the above water solubility test was a polymer aqueous solution having a concentration of 0.1% by weight. To this solution, sodium chloride was added in an amount corresponding to a concentration of 1M, and a viscosity (standard viscosity) was measured by a BL type viscometer at 25°C and a rotor rotational speed of 60 rpm using a BL adapter. The standard viscosity obtained by such a method is commonly used as a value having a correlation with molecular weight.

### Color tone

The polymer powder was visually observed to evaluate color tone of the polymer.

The evaluation results are set forth in Table 1.

**Table 1**

| | Acrylonitrile hydration catalyst | Additive | Solubility of polymer | Viscosity of polymer aqueous solution (mPa·s) | Color tone of polymer (visual observation) |
|---|---|---|---|---|---|
| Ex. 2 | nitrile hydratase | barbituric acid | BB | 5.6 | white |
| Ex. 3 | nitrile hydratase | dimedone | AA | 5.7 | white |
| Ex. 4 | nitrile hydratase | hydantoin | BB | 5.5 | white |
| Comp. Ex. 1 | nitrile hydratase | not added | DD | immeasurable* | light yellow |
| Comp. Ex. 2 | nitrile hydratase | malonic acid | (not polymerized) | (not polymerized) | (not polymerized) |
| Comp. Ex. 3 | copper catalyst | dimedone | CC | 5.5 | white |

| | | | | | |
|---|---|---|---|---|---|
| *: Viscosity of the filtrate was immeasurable because passing of the filtrate was slow and filtration was actually impossible. | | | | | |

## Claims

1. A process for producing acrylamide, the process comprising:
(i) hydrating acrylonitrile using:
a bacterial cell containing nitrile hydratase, or
a substance containing nitrile hydratase obtainable by treating a bacterial cell containing nitrile hydratase;
(ii) adding to the acrylamide at least one compound selected from dimedone, barbituric acid, hydantoin, and salts thereof.

2. The process according to claim 1, wherein the compound is selected from hydantoin and salts thereof.

3. A process for producing an acrylamide-based polymer, comprising:
(i) preparing acrylamide according to the process of claim 1 or claim 2;
(ii) homopolymerizing the acrylamide, or copolymerizing the acrylamide and at least one unsaturated monomer copolymerizable with the acrylamide.

## Patentansprüche

1. Verfahren zur Herstellung von Acrylamid, wobei das Verfahren Folgendes umfasst:
(i) Hydrieren von Acrylonitril unter Verwendung der Folgenden:
einer Bakterienzelle, die Nitrilhydratase enthält, oder
einer Substanz, die Nitrilhydratase enthält, die durch Behandeln einer Bakterienzelle, die Nitrilhydratase enthält, erhalten werden kann;
(ii) Zusetzen von zumindest einer Verbindung, die aus Dimedon, Barbitursäure, Hydantoin und Salzen davon ausgewählt ist, zu dem Acrylamid.

2. Verfahren nach Anspruch 1, wobei die Verbindung aus Hydantoin und Salzen davon ausgewählt ist.

3. Verfahren zur Herstellung eines Polymers auf Acrylamidbasis, das Folgendes umfasst:
(i) Herstellen von Acrylamid gemäß einem Verfahren nach Anspruch 1 oder Anspruch 2;
(ii) Homopolymerisieren des Acrylamids oder Copolymerisieren des Acrylamids und zumindest eines ungesättigten Monomers, das mit dem Acrylamid copolymerisierbar ist.

## Revendications

1. Procédé de production d'acrylamide, le procédé comprenant :
(i) l'hydratation d'acrylonitrile en utilisant :
une cellule bactérienne contenant une nitrile hydratase, ou une substance contenant une nitrile hydratase pouvant être obtenue en traitant une cellule bactérienne contenant une nitrile hydratase ;
(ii) l'ajout à l'acrylamide d'au moins un composé choisi parmi la dimédone, l'acide barbiturique, l'hydantoïne et les sels de tels composés.

2. Procédé selon la revendication 1, lequel composé est choisi parmi l'hydantoïne et les sels de ce composé.

3. Procédé de production d'un polymère à base d'acrylamide, comprenant :
(i) la préparation d'acrylamide selon le procédé de la revendication 1 ou de la revendication 2 ;
(ii) l'homopolymérisation de l'acrylamide, ou la copolymérisation de l'acrylamide et d'au moins un monomère insaturé copolymérisable avec l'acrylamide.
